(19) Europäisches Patentamt
European Patent Office
Office européen des brevets

(11) Publication number: 0 244 812 B1

(12) # EUROPEAN PATENT SPECIFICATION

(45) Date of publication of patent specification: 04.04.90

(51) Int. Cl.[5]: C 07 B 33/00, C 25 B 3/02, C 07 C 45/28, C 07 C 46/04, C 07 C 305/04

(21) Application number: 87106460.6

(22) Date of filing: 05.05.87

(54) **Oxidation of organic compounds.**

(30) Priority: 05.05.86 US 859548
05.05.86 US 859565
10.10.86 US 917462
10.10.86 US 917442
01.12.86 US 936521
17.12.86 US 942758

(43) Date of publication of application:
11.11.87 Bulletin 87/46

(45) Publication of the grant of the patent:
04.04.90 Bulletin 90/14

(84) Designated Contracting States:
AT BE CH DE ES FR GB GR IT LI LU NL SE

(56) References cited:
EP-A-0 027 400
CA-A-1 132 996
GB-A-1 192 037
US-A-3 671 552

(73) Proprietor: W.R. Grace & Co.-Conn. (a Connecticut corp.)
Grace Plaza 1114 Avenue of the Americas
New York New York 10036 (US)

(72) Inventor: Kreh, Robert Paul
8008 Cipher Row
Jessup Maryland 20794 (US)
Inventor: Spotnitz Robert Mark
1016 Hartmond Road
Catonsville Maryland 21228 (US)

(74) Representative: Abitz, Walter, Dr.-Ing. et al
Abitz, Morf, Gritschneder, Freiherr von Wittgenstein Postfach 86 01 09
D-8000 München 86 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 244 812 B1

Courier Press, Leamington Spa, England.

## Description

The present invention is directed to an improved electrochemical oxidation process for forming quinones and aromatic aldehydes or ketones from corresponding aromatic and alkyl aromatic compounds in good yields and high selectivity. More specifically, the invention described and claimed herein requires the use of a strong aqueous acid solution having high concentrations of ceric methanesulfonate and/or ceric trifluoromethanesulfonic acid dissolved therein or, alternately, requires the use of an aqueous-organic cosolvent system with lower concentrations of methanesulfonic acid therein. The invention is also directed to the use of thallium organosulfonate, in particular thallium methanesulfonate.

The quinones and aromatic aldehydes or ketones obtainable by the present process have a wide variety of known utility. For example, the quinones, such as naphthoquinone, are known additives in the paper making industry. The aldehydes, such as benzaldehyde, tolualdehyde and the like, and ketones, such as p-methylacetophenone, are known intermediates used in forming fragrance components useful in perfumes and colognes. Certain aldehydes and ketones have been used in forming pharmaceuticals.

The products achieved by the present invention have been previously formed by a variety of processes which may be generally classified as chemical or electrochemical. For example, aromatic aldehydes have been chemically formed by air oxidation conducted in an oxygen enriched environment at high temperatures and pressure in the presence of a transition metal catalyst or by using known chemical oxidizing agents which are not regenerable. Oxidation has also been achieved by direct electrochemical oxidation of aromatic compounds in the presence of dilute acid electrolytic solutions as described i U.S. Patents 4,298,438 and 4,354,904 and by indirect electrochemical oxidation in which the oxidant is electrolytically generated and, in turn, used to oxidize the aromatic compound.

Compounds which are known to be capable of acting as an indirect oxidant include transition metal salts, particularly the metals of cobalt, chromium, manganese, iron, lead, silver and cerium. Because regeneration of the spent metal to its higher oxidation state is not always highly effective and/or other insoluble salts, such as oxides, etc., are formed, those skilled in this art tend to use the salts of chromium, manganese, cobalt, iron or lead as these salts are less expensive and replacement of spent materials do not greatly detract from the economics of the process. However, each of these metal ion oxidants have certain properties which cause them to make the oxidation process ineffective. For example, chromium ions give poor selectivity towards the desired products, cerium and manganese salts are believed to have low solubility of the oxidized and/or reduced ions in acidic solutions causing reduced activity or separation problems, the higher oxidation states of silver, cobalt and lead ions are not very stable and, in the case of iron, is not very reactive. Indirect electrochemical oxidation has been further complicated by the properties of the anion specie present. For example, certain anions (e.g., chloride, nitrate, perchlorate) are highly reactive with the organic substrate producing by-prouducts or conditions which preclude their use on a commercial scale. Other less reactive anions (e.g., sulfate, acetate, fluoride, boron fluoride, silicon fluoride) generally form salts of low solubility, inhibit the rate of reaction of the oxidant with the organic substrate and/or inhibit the ability of the spent oxidant to be regenerated. In addition, certain organic acid salts (e.g., benzenesulfonate) have been found to be insufficiently stable to be useful in an indirect oxidation process.

Ceric ion is a well known oxidizing agent in organic chemistry. It has the potential of presenting an excellent one electron but has not been previously used extensively or on an industrial scale because of the inability of both the ceric and cerous ions to be maintained in solution at high concentrations and, preferably, under high acidity causing its use to be limited to slurries or very low concentrations. In addition, ceric oxidant has been associated with poor reactivity and selectivity. The cerium salts are prohibitively expensive and must, therefore, be capable of being stable, react with the organic substrate cleanly and be easily regenerated to its higher valence state. This requires the ceric salt to exhibit a high degree of stability and solubility in the electrolyte solution and be capable of achieving good reaction rates. In addition, the cerous ion must also be highly soluble to be capable of being regenerated to the ceric ion under conditions of high current efficiency at the anodic portion of the electrochemical cell. However, conditions (i.e. high acidity) preferred for best utilization of the ceric ion have previously been believed as being counterproductive to achieving proper conditions for cerous salt utilization. Therefore, it has heretofore been believed necessary to use the cerium salt at very low concentrations and under a very narrow set of conditions including those which could not demonstrate the potential necessary to provide an effective industrially suitable process.

Canadian Patent 1,132,996 to Oehr describes a process for oxidizing naphthalene to naphthaquinone using ceric sulfate in dilute sulfuric acid. Both cerous sulfate and ceric sulfate are known to have low solubility in dilute acid [Solubilities of Inorganic and Organic Compounds, Vol. 3, Part I, Ed. by H. L. Silcock (1974)] and the solubility decreases with increasing acid concentration. The solubility limitations lead to the use of inefficient slurry conditions or to the need for large volumes of solution to oxidize small quantities of the organic compound. Similar problems are encountered with other salts of low solubility.

European Patent Application 0075828 of Mayeda et al describes a process for oxidizing fused ring compounds to their respective quinones using ceric nitrate in dilute nitric acid. Although solubility does not present a problem, the nitrate anion is known to react with the organic reactant forming nitrogen containing by-products which are difficult to handle and remove. Cerium salt solutions containing

perchlorate anions have also been disclosed as a useful oxidant [Prospects for the Indirect Electrolytic Oxidation of Organics, by N. Ibl et al., AIChE Symposium Series, Electroorganic Synthesis Technology, Pg. 45, (1979)] but it is well known that the perchlorate reacts explosively with organic materials and, therefore, is unsuitable for commercial scale processes.

M. Marrocco et al [J. Org. Chem., Vol. 48, No. 9, Pg. 1487 (1983)] conducted a study of the oxidation of an organic substrate by various cerium salts in different acid electrolytes. Each of the cerium salt systems contained excess perchlorate or trifluoroacetate anions and the cerium ions were maintained at very low concentrations. Even at the low concentrations, many of the systems were slurries and conversion and selectivity were low. Several systems, including cerium perchlorate or trifluoroacetate in methanesulfonic acid, were shown to be ineffective.

It must be understood that although cerous/ceric ions have been known and used in oxidation reactions, there is a need to have a system wherein the ceric oxidant can be sufficiently stable under oxidizing conditions to be useful in indirect electrochemical processes, to be capable of undergoing repeated cycling between its cerous ($Ce^{+3}$) and ceric ($Ce^{+4}$) species in a high degree of efficiency under the reaction and electrolysis conditions, to be highly selective in forming the desired carbonyl group containing compounds, to be capable of exhibiting high reaction rates to make the process attractive on a commercial scale, to have high solubility to aid in the efficiency of the reaction and to eliminate the problems associated with slurries of cerium salts. It is readily seen that a means of achieving this combination of desired properties would aid in providing a process which would find a high degree of acceptance in electrochemical oxidation of aromatic and alkyl substituted aromatic compounds.

The present invention is also directed to an improved electrochemical process for oxidizing organic compounds using an organicsulfonic acid solution having certain thallium(III) organosulfonates dissolved therein as fully described hereinbelow.

Thallium is a known oxidizing agent which has the potential of presenting an excellent two electron oxidant but has not been previously used to an extensive degree or on an industrial scale because of the inability of both the thallium(I) and thallium(III) species to be maintained in solution at high concentrations and due to the difficulty of generating the thallic oxidant in a simple and effective manner. For example, U.S. Patent 3,048,636 teaches the use of low concentrations of thallium sulfate in order to avoid precipitation of either thallium(III) oxides, thallium(I) sulfate or complexes formed from thallium(I) and (III) sulfate. Thallium sulfates are generally restricted to low concentrations or must be used as a slurry. Both conditions are associated with poor reactivity and selectivity. One of the few thallium salts which exhibits high solubility is thallium(III) perchlorate. However, a potentially explosive situation is formed when the perchlorate anions are placed in contact with organic compounds.

The thallium salts as with the cerium salts discussed above are prohibitively expensive and must, therefore, be capable of being stable, react with the organic substrate cleanly and be easily regenerated to its higher valence state. This requires the thallium(III) salt to be capable of exhibiting the same properties described above to achieve an effective oxidant material. It has heretofore been believed that thallium must be used under a very narrow set of conditions or under inefficient conditions which could not demonstrate the potential necessary to provide an effective industrially suitable process.

Various processes are known to generate thallic ions from thallous ions but the majority of them are either expensive to do, require additional oxidant which precludes the systems use in providing a clean organic synthesis, causes accumulation of undesirable by-products, has a low efficiency of ability to generate the thallic ion or a combination of these defects. For example, chemical oxidation of thallous is, of course, possible with the very powerful agents such as chlorine gas and aqua regia, but these materials are objectionable as being somewhat difficult to handle (requiring expensive low-corrosion equipment), and cause the accumulation of undesirable materials in the system.

Hirose et al., in U.S. Patent No. 3,399,956, report a system for oxidizing thallium with oxygen, which involves an acidic aqueous medium containing chloride or bromide and an ion of a "redox metal" such as copper or iron. In U.S. Patent 3,479,262, MacLean et al describe a process to oxidize an olefin using thallium. The thallic ion is regenerated by a noble metal catalyzed oxidation using cerium(IV) as the oxidizing agent.

Other systems for oxidizing thallium are described in U.S. 3,486,992 to Frye, U.S. 3,759,804 to LeBris et al., U.S. 4,031,196 to Lenard, U.S. 4,115 to Brill, U.S. 4,115,421 to Brill, U.S. 4,058,592 to Rizkalla, U.S. 4,371,431 to Switzer et al as well as other methods.

Each of the above processes of forming thallium(III) has one or more disadvantage which makes it inappropriate for use in being an effective oxidant for organic compounds. In most instances either the thallous or the thallic specie is insoluble in the reaction medium. When the thallous specie is insoluble it impedes the separation and recovery of the organic product as well as lowering the effective oxidation to thallic ions. When the thallic specie is of low solubility, it reduces its effectiveness as an oxidant for the organic substrate.

It must be understood that although cerous/ceric ions and thallous/thallic ions have been known and used in oxidation reactions, there is a need to have a system wherein the oxidant can be sufficiently stable under oxidizing conditions to be useful in indirect electrochemical processes, to be capable of undergoing repeated cycling between its reduced ($Ce^{+3}$) ($Tl^{+1}$) and oxidized ($Ce^{+4}$) ($Tl^{+3}$) species in a high degree of efficiency under the reaction and electrolysis conditions, to be capable of exhibiting high reaction rates to

make the process attractive on a commercial scale, to have high solubility to aid in the efficiency of the reaction and to eliminate the problems associated with slurries of thallium salts. It is readily seen that a means of achieving this combination of desired properties would aid in providing a process which would find a high degree of acceptance in electrochemical oxidation of organic compounds.

The present invention is also directed to a novel, solid cerium(IV) product and to its utility as an oxidant of aromatic and alkyl substituted aromatic compounds to their corresponding carbonyl group containing compounds. More specifically the solid product described and claimed herein is a cerium methanesulfonate hydroxide hydrate.

None of the previously known cerium (IV) compounds is ideal for the purpose of achieving fast, selective oxidation of an aromatic compound. As discussed above, anions of certain cerium salts are highly reactive, exhibit unstable conditions which preclude their use on a commercial scale or are composed of less reactive anions which inhibit the rate of reaction and/or inhibit the ability of the spent oxidant to be readily regenerated.

There is a need for a ceric oxidant which is capable of exhibiting high reaction rates to make the process in which they are used commercially attractive, is a solid stable material capable of being easily transported, stored and metered in necessary quantities and is capable of readily dissolving in water to provide an aqueous solution.

## Summary of the Invention

The present invention is directed to an electrochemical process wherein divalent cerium and thallium are generated to their respective higher oxidation state (ceric; thallic) and used as an oxidant of certain organic compounds in good selectivity. One embodiment of the present process requires the utilization of at least 0.2 molar concentration of cerium salts of methanesulfonic acid or of trifluoromethanesulfonic acid dissolved in a highly acidic aqueous solution containing a substantial excess of the respective salt forming free acid. In another embodiment, the methanesulfonic acid solution may contain lower amounts of free acid provided the system contains an organic cosolvent which exhibits at least 2% solubility in water. In a still further embodiment, the process requires the utilization of at least 0.1 molar concentration of thallium salts of certain organosulfonic acids dissolved in an acidic solution containing an excess of at least 1 Normality corresponding free organosulfonic acid. The specific oxidizing solutions, as described hereinbelow, exhibit the desired combination of properties (stability, solubility, reactivity, capability to achieve high current density, capability of repeated cycling in an efficient manner, and selectivity of product formation) to provide a commercially attractive process.

The present invention is also directed to a solid, stable product, ceric methanesulfonate hydroxide hydrate, as more fully described below, and to its use in the oxidation process of the present invention to transform aromatic and alkyl substituted aromatic compounds to carbonyl containing compounds in high selectivity.

## Detailed Description of the Invention

The present invention is directed to a process for selectively forming carbonyl containing compounds from respective aromatic compounds.

Certain terms used in the present specification and in the appended claims are defined herein below to aid in providing a clear description of the invention:

The term "aromatic" shall, unless specifically indicated otherwise, refer to benzylic and fused benzylic compounds such as benzene, naphthalene, anthracene and the like. The compounds may be unsubstituted or may contain substitution groups which are inert to oxidation such as halides, alkoxy, nitro, sulfonyl, amide, tertiary amino, tertiary alkyl and carboxylate ester groups.

The term "alkyl aromatic" refers to $C_1$—$C_6$ alkyl substituted benzylic and fused benzylic compounds. The compounds shall contain one or more than one primary or secondary $C_1$—$C_6$ alkyl group attached to the aromatic ring and may, in addition, contain groups which are inert to oxidation such as halides, alkoxy, nitro, sulfonyl, amido, tertiary amino, tertiary alkyl, and carboxylic ester groups. Examples of such compounds include toluene, (o, m or p) xylene, trimethylbenzene, (o, m or p) ethyltoluene, (o, m or p) propyltoluene, (o, m or p) methoxyethylbenzene, (o, m or p) ethoxyethylbenzene, 1,2-dimethylnaphthalene, (o, m or p) methyl-N,N-dimethylaniline (o, m or p) chlorotoluene and the like.

The term "indirect electrochemical oxidation" refers to an oxidation of an aromatic or alkyl aromatic compound which proceeds in two steps such that the first step provides a metal ion oxidant (e.g. $Ce^{+4}$ or $Tl^{+3}$) by anodic charge exchange and the second step comprises the reacting of the metal ion oxidant with an aromatic or alkyl aromatic compound to produce carbonyl containing compounds. The oxidation of the aromatic or alkyl aromatic compound does not occur selectively in the absence of the metal ion oxidant. The indirect electrochemical oxidation of the organic substrate can be conducted in the electrochemical reactor (in-cell) or in a separate reactor (ex-cell).

The terms "cerous", "ceric" and "cerium" refer, respectively to the cerium ion or salt of a cerium ion in its lower valence state (+3), its higher valence state (+4) and as a mixture of both lower and higher valence state species.

The terms "thallous", "thallic" and "thallium" refer, respectively to the ion or salt of a thallium in its

lower valence state ($Tl^{+1}$), its higher valence state ($Tl^{+3}$) and as a mixture of both lower and higher valence species.

The term "system" refers to the water and organic cosolvents. The organic cosolvent must have at least 2 percent solubility in water at the process conditions.

The term "organosulfonic acid" and "organosulfonates" as used herein and in the appended claims with respect to the thallium oxidation shall refer to the free acid and salts of the free acid, respectively, wherein the acid has the general formula

$$RSO_3H$$

in which R represents methyl, trifluoromethyl, benzyl or tolyl group. The subject term shall include each individual acid or mixtures thereof. The preferred organosulfonic acid is methanesulfonic acid.

The terms "chromium cations" shall refer to chromium ions or salts of chromium ions in any of its plus two, plus three and plus six valence states as well as mixtures of said species.

The term "quinone" and "quinonoid" as used herein and in the appended claims refers to cyclic diones. These compounds can be viewed as having two C=O groups in which each carbon is part of the cyclic ring structure. The diones can be positioned ortho or para to each other and include, for example, para-benzoquinone, ortho-benzoquinone, 1,2-, 1,4- and 2,6-naphthaquinone, acenaphthaquinone, anthraquinone and the like.

The present invention provides an improved indirect electrochemical oxidation process wherein certain solutions of cerium or thallium are utilized.

The utilization of the presently required solution unexpectedly provides the combination of advantages of:

(1) high solubility of both the cerous and the ceric ions over a wide acid concentration provided the required minimum acid concentration is maintained;

(2) high current efficiency at high current density (of at least about 75 $mA/cm^2$ or greater) to provide effective anodic oxidation of the cerous ions to ceric ions;

(3) fast reaction rate of the ceric oxidant with the organic reactant;

(4) high selectivity of the oxidation of the organic reactant to formation of desired carbonyl containing compounds;

(5) passivity of the anion and the free acid to the organic reactant and to the electrodes of the cell; and

(6) clean, uncomplicated reduction at the cathode to again aid in effecting an efficient process.

First Embodiment

In one embodiment of the present invention, the improvement requires the utilization of cerium salts of methanesulfonic acid or trifluoromethanesulfonic acid present in concentration of at least 0.2 molarity in an aqueous solution of the free acid. The free acid concentration methanesulfonic acid should be 1.5 to 9 Normality and for trifluoromethane sulfonic acid should be 0.75 to 7 Normality to provide efficient reaction rates for oxidizing the organic substrate and high current efficiency to regenerate the oxidant.

It has now been unexpectedly found that certain cerium salts can be used as an effective oxidant for indirect electrochemical synthesis when used according to the present invention. The cerium salt of methanesulfonic acid should be in a (CeMS) solution which contains at least 1.5 and preferably at least 2 Normal free acid in the solution. The cerium salt of trifluoromethanesulfonic acid ($CeF_3MS$) should be in a solution which contains at least 0.75 and preferably at least 1.5 Normal free acid in the solution. The solution must have the cerium salts substantially completely dissolved in the solution and the combined cerium ion concentration to be at least 0.2 molar.

It is disclosed herein that both the cerous and ceric methanesulfonate salts unexpectedly exhibit good solubility when the solution contains at least 1.5 molar concentration of free methanesulfonic acid in the aqueous medium. At low concentrations of free methanesulfonic acid, the solubility of the cerium (IV) salt decreases markedly. The use of cerium (IV) methanesulfonate slurries at low acid concentration has beem associated with low reaction rates and poor selectivity to the carbonyl derivative of the aromatic and alkyl aromatic substrate.

The subject process requires the use of certain cerium salts. Solutions of the salts can be readily formed by reacting a cerous salt of an inorganic acid with aqueous solution of the free methanesulfonic or trifluoromethanesulfonic acid. The resulting aqueous solution should, preferably, be substantially free of extraneous anions of other acids such as sulfates, nitrate, perchlorate, halide, acetate, trifluoroacetate and the like. It is preferred that the concentration of such extraneous anions be maintained at a low value of from 0 to 0.5 preferably from 0 to 0.1 mole per mole of cerium ions present in the solution. It is therefore most desired to form the subject salts from cerous carbonate, cerium dioxide, cerium hydroxide and the like and most preferably from cerous carbonate. When other inorganic acid salts are used, their anions should be substantially removed from the solution by known means prior to using solution in the subject process. For example, if sulfate ions are present they can be removed by precipitation with lead(II) carbonate. Similarly, chloride ions can be removed by treating the solution with silver carbonate. Other extraneous ions can be removed in similar manners known in the art.

As discussed above, various cerium salts have been proposed as an oxidant in electrochemical

oxidation processes. The salts have been either formed from reactive anions or from a more passive anion in which case the salt is normally present in the form of a slurry or as a very dilute solution due to solubility restrictions attributable to either one or both metal ions (e.g. $Ce^{+3}$, $Ce^{+4}$) salt form specie. The present invention unexpectedly provides a means of maintaining high concentrations of both the ceric and cerous species in solution and thus permits cyclical formation of the cerium ions without formation of insoluble material.

The process requires the electrolytic solution of CeMS to contain free methanesulfonic acid in at least 1.5 molar concentration, normally from 2 to 9 molar, preferably from 2.5 to 8 molar and most preferably from 2.5 to 7 molar concentration. When $CeF_3MS$ is used the present process requires the electrolytic solution to contain free trifluoromethanesulfonic acid in at least 0.75 molar concentration, normally from 1.5 to 7 molar, preferably from 1.5 to 6 molar and most preferably from 2 to 5 molar concentration. Further, it is preferable that the electrolytic solution be substantially free of inorganic acids although small amounts as discussed above may be present.

The ceric and cerous salts can be dissolved in the presently described solution at high concentrations without causing precipitation of either one of the salt species. The solution can have a combined concentration of ceric and cerous metal ions at levels of 0.2 molar or greater under the process temperature conditions. Cerium concentrations of 0.5 molar and 1 molar and greater can be achieved when the preferred and most preferred electrolytic solutions are used. It is realized that under the present process, the cerium ions can be maintained in solution at concentrations which are higher or less than the above stated concentrations provided they are maintained in solution. The specific concentration which meet economic, process and solubility restraints can be readily determined by conventional techniques.

The organic compounds which can be effectively oxidized using the solution of the present process are aromatic and alkyl aromatic compounds. The aromatic compounds include benzylic and fused benzylic ring compounds which may be unsubstituted or be substituted with a group which is substantially inert to to oxidation. Examples of such compounds include benzene, naphthalene, anthracene and the like as well as such compounds which contain groups attached to the ring which are inert to the present indirect oxidation. Such groups can be readily determined by simple laboratory testing and include ($C_1$—$C_4$) alkoxy, tert-alkyl ($C_4$—$C_7$), phenoxy, nitro, tertiary amino, sulfonyl, amido, and carboxylic acid ester groups and the like. The alkyl substituted aromatic compounds include the above defined aromatic compounds which further contains at least one primary alkyl or secondary alkyl group or both.

The aromatic and alkyl aromatic organic compounds described above are oxidized to their respective carbonyl containing compounds by contacting the organic compound with the acidic aqueous solution or system described above which contains the ceric oxidant. The contacting of the oxidant and the organic compound may be conducted directly within the electrolytic cell. However, it is preferable to transfer the subject oxidant containing solution to a separate reactor vessel where it is contacted with the organic compound to be oxized under agitation. The organic compound can be introduced into the reactor either dissolved or dispersed in the aqueous phase or dissolved in a co-solvent with the aqueous solution.

It has been unexpectedly found that the solution used in the present process is capable of providing ceric ions in high concentration and at high solubility in the liquid phase to provide high reaction rate in oxidizing the aromatic and alkyl aromatic organic compound and to have high solubility of the spent cerium(III) permitting easy separation and recovery of the oxidized product. In addition, the subject process unexpectedly provides a means for readily and selectively forming quinones (from aromatic compounds) and aldehydes or ketones (from alkyl aromatics) without substantial by-product formation. When the organic compound is present in excess, such as from about three to ten fold excess of stoichiometry, one will form alcohol as well as the above quinone, aldehyde or ketone.

The present process further provides a means of readily converting certain aromatic and alkyl aromatic compounds to their corresponding carbonyl containing compound which was either difficult or impractical to accomplish by prior use of ceric salts. This ability is probably due to a combination of factors (although not meant to be a limitation of the present invention) capable of being used under the present invention. For example, the ability to maintain high acid normality of the solution may enhance oxidizing certain organic substrates. The high concentration of the oxidant in solution may catalyze the conversion of certain organic substrates to desired carbonyl containing compounds.

The aqueous solution may contain an organic co-solvent which can aid in solvating the aromatic or alkyl aromatic reactant. The co-solvent may be miscible or immiscible with the aqueous phase. Such co-solvents may be any which is inert in the system as are well known and include lower alkyl alcohols such as methanol, ethanol, isopropanol and the like, acetonitrile and the like. Other conventional materials may be added to the system provided they are inert to the cerium salt and free acid used herein. Examples of such materials include anionic surfactants such as sodium dodecylbenzene sulfonate and the like and cationic surfactants such as tetrabutylammonium hydroxide and the like.

Second Embodiment

According to a second embodiment of the subject invention, the cerous salt of methansulfonic acid can be dissolved in an aqueous system at high concentrations without causing precipitation by incorporating an organic cosolvent therein. Effective cerium solutions can be achieved when the system contains low

concentrations of from 0 to about 1.3 molar of free methanesulfonic acid in excess of that needed to form cerious methanesulfonate salt by incorporating an organic cosolvent as fully described herein below in the electrolytic solution. Although the cerium (IV) salt is substantially insoluble in the system at the low acidcid concentration, it has been unexpectedly found that the organic reactant is capable of being rapidly oxidizedzed by the ceric specie present in the system. The total concentration of cerium present in the systemem (dissolved and dispersed) should be at least 0.2 molar with higher concentrations of at least 0.5 beinging preferred and at least 1 molar being most preferred. The specific concentration which meets economic,nic, process and solubility restraints can be readily determined by conventional technique.

The organic cosolvent of the present system must have a solubility with water which is at least 2 percent (on a weight-to-weight basis) and preferably at least 5 percent. The organic cosolvent must be substantially inert under the conditions encountered in the present process. The cosolvent must, therefore, be substantially inert with respect to the acidic and redox conditions of the process. Compounds which are most suitable as the organic cosolvent include nitriles such as acetonitrile, propionitrile, butyronitrile and the like; nitroalkanes such as nitromethane and nitroethane; alkoxy compounds such as dimethyl ether, diethyl ether, methylene dimethyl ether, methylene diethyl ether, tetrahydrofuran, 1,2-dimethoxyethane and the like; sulfonyl containing compounds such as dimethyl sulfone, tetramethyl sulfone (sulfolane), diethyl sulfone and the like; sulfoxides, such as dimethyl sulfoxide and the like; amides such as N-methyl pyrrolidone, dimethylformamide, 2-pyrrolidone and the like; organic carbonates, such as propylene carbonate and the like; alcohols such as methanol, ethanol, t-butanol and the like; ketones such as acetone, methyl ethyl ketone and the like; their equivalents as well as mixtures thereof. The most preferred organic cosolvents are $C_1$—$C_3$ alkyl nitriles, especially acetonitrile, and $C_1$—$C_2$ nitroalkanes. The organic cosolvent can be present as part of the system in from 2 percent to about 50 percent. It can be introduced into the system as part of the aromatic or alkyl aromatic feed (especially in a batch process) or separately to form the liquid system of the process.

The aqueous system may contain one or a mixture of organic cosolvents. In addition, other conventional materials may be added to the system provided they are inert to the cerium salt and the acid contained therein. Examples of such materials include conventional anionic, cationic or nonionic surfactants.

The cerium (IV) species may be generated by electrolysis and has been found to be a good one electronn oxidant which can rapidly and effectively oxidize aromatic and alkyl aromatic compounds to theirir respective carbonyl compounds in high yield and selectivity. The present process accomplishes thisis desired result even though within this embodiment the cerium(IV) specie is a solid dispersed in the system.n. The process may be carried out as a single chemical oxidation or as a mediated electrochemical process.s. The mediated process is preferred as it permits the use and reuse of the cerium methanesulfonate.

The oxidation of organic substrates using ceric oxidant as described herein above with respect to the first and second embodiments of the subject invention has been found to be enhanced by the presence of a catalytic amount of chromium cations. Although chromium itself has been used as an oxidant because of its mutlivalence states, it is known as a very ineffective oxidant and the enhanced results presently achieved can not be attributed to the additive effective of the ceric and chromium cations present in the present system. The presence of catalytic amounts of chromium cations to a ceric oxidant has been unexpectedly found to provide enhanced yield and/or selectivity of the quinone product. When alkyl substituted fused ring systems are used as the organic substrate, the yield of the preferred quinone is normally enhanced when formed according to the present invention. For example, 2-methylnapthalene yields 2-methylnaphthaquinone in substantially higher yields using the present process than is achievable when using only the same cerium salt alone. It is believed that alkyl substituted aromatics provide greater yields of the related quinone derivative over the aldehyde or ketone derivative.

The chromium cation can be introduced by the presence of any chromium salt which is soluble in the acidic solution of the process. Such salts include the chromium oxides, including the mono, di and trioxides, sulfate, nitrate, halides, in particular the chloride and bromide, acetate, phosphate and the like.

The chromium cation can be introduced in any of its positive valencies which are the plus two, plus three or plus six valence state. The chromium cation should be present in the system in a catalytic amount with respect to the cerium ions present in the system. The chromium to cerium molar ratio can range from about 1:600 to 1:20 with a range of from 1:150 to 1:30 being preferred. The presence of large amounts of chromium in the system should be avoided as the yield of quinone product is drastically reduced under such conditions.

## Third Embodiment

It has now been unexpectedly found that thallium organosulfonates can be used as effective oxidants for indirect electrochemical synthesis when used according to the present invention which requires the solution to contain an excess of organosulfonic acid corresponding to salt used. The excess should preferably be at least about 1 and most preferably from 1 to about 9 Normal free acid (with respect to thallous) in the solution, to have the thallium salts substantially completely dissolved in the solution and to have the combined thallium ion concentration be at least 0.1 molar. The utilization of the presently required solution unexpectedly provides the combination of advantages discussed above with respect to the preferred cerium oxidation although high selectivity is not always achieved.

The subject process requires the use of the salts of thallium organosulfonate, most preferably thallium methanesulfonate. Solutions of the salts can be readily formed by reacting a thallium salt of an inorganic acid with aqueous solution of the organosulfonic acid in the same manner and concentrations as described above for forming cerium methanesulfonate.

The present invention unexpectedly provides a means of maintaining high concentrations of both the thallic and thallous species in solution and thus permits cyclical formation of the ions without formation of insoluble material. The present process requires the electrolytic solution to contain certain free organosulfonic acids in at least 0.1 molar concentration, normally from 1 to 9 molar and preferably from 1.5 to 8 molar concentration. Further, it is preferable that the electrolytic solution be substantially free of inorganic acids although small amounts as described above may be present.

The solution in which the process of the present invention is conducted can be aqueous solution containing the above described concentrations of thallium organosulfonate and of the corresponding free organosulfonic acid. Alternately, the subject process of transforming the thallous ions to thallic ions and using the latter as an oxidant for an organic substrate can be conducted in solutions formed with an organic polar liquid. The organic polar compound should be liquid under the reaction process conditions and should be substantially inert with respect to oxidation by the thallic ion present and substantially inert with respect to the organic substrate. These properties can be readily determined by simple tests. Suitable liquids include compounds containing one or more groups selected from nitriles, alcohols, amides, ethers, and nitro groups and mixtures thereof. In addition, the process can be carried out in an alkanesulfonic acid. Examples of suitable nitriles include alkyl nitriles wherein the alkyl is a $C_1$—$C_{10}$, preferably a $C_1$—$C_5$ alkyl group such as acetonitrile propionitrile, butyronitrile and the like. Examples of suitable hydroxyl containing compounds include $C_1$—$C_{12}$ alkyl alcohols such as methanol, ethanol, butanol, dodecanol, ethylene glycol and the like. Examples of suitable amides include alkylamides such as N,N-dimethylformamide pyrrolidone and the like. Examples of suitable ethers include dialkyl ethers, polyethers and alkyl aryl ethers such as ethyl ether, methyl ethyl ether, phenyl ether, ethyl phenyl ether, diglyme and the like. The nitro compounds which are suitable include nitroparafins such as nitromethane, nitroethane, 1-nitropropane, 2-nitropropane and the like. The above described polar organic liquids can be used in combination with water or can be used separately as the reaction medium.

The process can also be carried out neat in an alkyl sulfonic acid such as with only excess methanesulfonic acid or the like.

The thallic and thallous salts can be dissolved in the presently described solution at high concentrations without causing precipitation of either one of the salt species. The solution can have a combined concentration of thallic and thallous metal ions at levels of 0.1 molar or greater under the process temperature conditions. Thallium concentrations of 0.5 molar and greater can be readily achieved. It is realized that under the present process, the thallium ions can be maintained in solution at concentrations which are higher or less than the above stated concentrations provided they are maintained in solution. The specific concentration which meet economic process and solubility restraints can be readily determined by conventional techniques.

Organic compounds which can be reacted with thallium(III) include those which have an index of hydrogen deficiency greater than zero. This index is described by J. B. Hendrickson, D. J. Cram and G. S. Hammond, Organic Chemistry, Third Edition, McGraw-Hill, Inc., 1970, at pages 72—73 and 82—83, as the number of pairs of hydrogen atoms which must be removed from a saturated alkane to give the empirical formula of a subject compound. For a hydrocarbon, then, the index represents the total of the rings and multiple bonds in a molecule. For compounds containing heteroatoms, the following principles can be used to make the index application: (1) oxygen and sulfur atoms do not change the index; (2) each halogen atom is equivalent to one-half of a hydrogen atom pair; and (3) each nitrogen atom requires that the "reference" saturated alkane be considered as having one extra hydrogen atom (i.e., a formula of $C_nH_{2n+3}$).

It is known that olefins can be oxidized with thallium to form carbonyl compounds and glycols (Grinstead, J. Org. Chem. Vol. 26, Pg. 238—240, 1961; and P. M. Henry, Homogeneous Catalysis, ACS Advances in Chemistry Series, Vol. 70, Pg, 126—154, 1968). Ethylene is oxidized to ethylene glycol and acetaldehyde. Kruse and Bednarski have reported in J. Org. Chem. Vol. 36, Pg. 1154—1155 (1971) that the oxidation can be stopped under certain conditions to provide the corresponding epoxide. For example, under weakly solvating conditions, propylene forms propylene oxide in good yields.

In addition to the oxidation of olefins, many other organic reactions occur with thallium. A review by R. J. Quellette, "Oxidation by Thallium(III)," Chapter 3 of Oxidation in Organic Chemistry, Part B, W. S. Trahanovsky, Ed., Academic Press, 1973, discusses work which has been done in the oxidation of steroids, oxidative rearrangement of chalcones, oxidative cleavage of cycloalkanes, oxidation of carbonyl compounds, oxidation of phenols, and the conversion of benzene to phenol.

The aqueous solution may contain an organic co-solvent which can aid in solvating the organic reactant. The co-solvent may be miscible or immiscible with the aqueous phase. Such co-solvents may be any which is inert in the system and can be selected from the classes of polar organic liquids discussed above. Other conventional materials may be added to the system provided they are inert to the thallium salt and free acid used herein. Examples of such materials include anionic surfactants such as sodium dodecylbenzene sulfonate and the like and cationic surfactants such as tetrabutylammonium hydroxide and the like.

The generation and subsequent regeneration of oxidant can be readily carried out by supplying the solution or system of the present invention to an electrolytic cell in either a batch or continuous manner. The cell may be either undivided or divided by a porous partition wall or membrane between electrodes. The electrodes may be of any suitable form such as plates, lattices, expanded metal, or reticulated porous material and the like. The anode may be any of the known materials suitable for preforming the metal-ion oxidation and are, preferably selected from lead, lead oxide, platinum, platinized titanium, platinized niobium or metal oxide-titanium composite. The cathode of the cell may be any of the known maerials suitable for performing reductions in the aqueous-acid solutions with or without the presence of metal ions such as, for example, steel, copper, and nickel. The use of the presently described cerium or thallium salt solution or system has, as one of its unexpected properties, the ability to readily and effectively generate and regenerate ceric or thallic oxidant from cerous or thallous ions, respectively, at high current density. Another unexpected property is the ability of the solution to cause a clean cathodic reduction without production of by-products which detract from the process and require separation therefrom. The electrolysis can be performed at voltages ranging from about 2 to 20 volts with current density ranging between about 0.1 to about 500 mA/cm$^2$, preferably from 10 to 400 mA.cm$^2$ and most preferably from 30 to 300 mA/cm$^2$ (based on electrode area excluding roughness factor). The electrolysis may be conducted at a temperature of from about −20 to 150°C and preferably from 0 to 100°C. It is most preferable to have the cell temperature and the reaction temperature (where the cell and chemical reactor are separate) be substantially the same.

The organic oxidation can be carried out under ambient temperature and pressure conditions. The temperature may be varied from about 0 to about 100°C with from 20 to 75°C being preferred. The pressure may be elevated or reduced for process reasons.

The solution or system removed from the reaction zone contains product and spent metal ion oxidant. The product can be readily separated from the solution by phase-separation, distillation, precipitation or extraction with an appropriate solvent such as alkanes acyclic or cyclic haloalkanes, dichloroalkanes, cyclohexane and the like. The particular mode of separation will depend upon the identity of the product formed and can be readily ascertained by the artisan.

The resultant solution (after separation of the product) will contain reduced salt as the sole or major component and may contain small amounts of unreacted oxidant metal salt. This solution can be returned to the electrolytic cell for regeneration of the metal ion oxidant. It has been found that the ceric/cerous and thallic/thallous salts used herein readily regenerate a multiplicity of cycles without formation of by-products which have detrimental effect on the efficiency of the process.

Fourth Embodiment

The ceric oxidant product of the subject invention is a solid compound which shall be labeled ceric methanesulfonate hydroxide hydrate. The compound is represented by the atomic formula $CeC_2H_{10}S_2O_9$ which may translate into $Ce(CH_3SO_3)_2O\ 2H_2O$ or $Ce(CH_3SO_3)_2(OH)_2\ H_2O$. Additional water may be associated with the compound. The solid has an elemental analysis of 36.2 wt. % (theory 36.65 wt. %) cerium, 6.6 wt. % (6.3 wt. %) carbon, 17.0 wt. % (16.75 wt. %) sulfur, 2.6 wt. % (2.62 wt. %) hydrogen and 37.6 wt. % (37.7 wt. %) oxygen. The compound exhibits (via differential scanning calorimetry) the onset of the melting slope at 195.6°C with a peak maximum at 215.5°C. Infrared spectral analysis (done with KBr pellet) of the subject compound is shown in Figure 1.

The subject ceric compound of the present invention can be formed by contacting certain cerous salts with methanesulfonic acid to provide the cerous methanesulfonate intermediate, electrolytically oxidizing the intermediate in the presence of a small excess of free acid and recovering the resultant ceric methanesulfonate hydroxide hydrate. Specifically, the ceric compound is formed by contacting, in an aqueous solution, a cerous oxide, carbonate, hydroxide or mixtures thereof with a small excess of free methanesulfonic acid over that required to neutralize the initial cerous salt. Therefore, for each mole of cerous salt (i.e., cerous carbonate, etc.) the solution should contain greater than three moles of free acid. The excess amount of acid should be small, such as from about 0.1 to 1.3 molar excess and preferably from about 0.5 to about 1.3 molar excess of free acid, over the stoichiometric amount required to form the cerous methanesulfonate. The concentration of cerous ions in the solution should be equal to or greater than about 0.2 molar and preferably from about 0.5 to 1.5 molar. Further, the solution should be free of extraneous anions of other inorganic acids such as sulfate, nitrate, perchlorate and the like. When such anions are present they should be removed by known means prior to using the solution to recover the subject compound.

The solution so formed is introduced into an electrolytic cell which may be either undivided or preferably divided by a conventional porous partition wall or membrane between electrodes. The electrodes may be of a form and material as described herein above. The cerous solution is introduced into the anolyte portion of a partitioned cell. The electrolytic cell provides an excellent means of permitting oxidation of cerous to ceric ions which combines with two moles of methanesulfonic acid to produce under the conditions described herein the present solid product, ceric methanesulfonate hydroxide hydrate.

The electrolysis can be performed at voltages ranging from about 2 to 20 volts with current density ranging between about 0.1 to about 500 mA/cm$^2$, preferably from 10 to 400 mA/cm$^2$ and most preferably from 30 to 300 mA/cm$^2$ (based on electrode area excluding roughness factor). The electrolysis may be

conducted at a temperature of from about −20 to 150°C and preferably from 0 to 100°C.

The above described weakly acidic cerous methanesulfonate solution has, upon electrolysis oxidation, been found to yield a precipitate product of the desired ceric methanesulfonate hydroxide hydrate. The ceric compound can be readily recovered by filtration or the like and washing with water, acetonitrile or other non-solvent liquids.

Solutions of the ceric compound can be readily formed by dissolving the compound in water. Although the subject compound is substantially insoluble in weakly acidic aqueous solutions (such as solutions having from 0.1 to 1.3 molar concentration of methanesulfonic acid), it has been surprisingly found that the compound is very highly soluble in substantially neutral (pH 6.5—7.5) or diionized water. Thus, aqueous solutions of the ceric methanesulfonate hydroxide hydrate can be readily formed by dissolving the subject ceric compound in substantially neutral water. Oxidation of organic compounds can be carried out in strong acidic solutions, that is, solutions having a concentration of at least 1.5 molar free methanesulfonic acid as described herein above. These solutions can be formed by rapidly adding the necessary amount of acid to the ceric compound-water solution to provide the required at least 1.5 molar acid concentration.

The following examples are given for illustrative purposes only and are not meant to be a limitation on the present invention as defined by the claims appended hereto. All parts and percentages are by weight unless otherwise indicated.

Example A-1

240 parts of methanesulfonic acid was added slowly to a stirred suspension of 53 parts of cerium(III) carbonate (obtained as pentahydrate) in 160 parts of water. Upon completion of evolution of carbon dioxide the resultant solution contained 0.6M cerous methanesulfonate [$Ce(CH_3SO_3)_3$] and 6M of free methanesulfonic acid.

The above solution (320 ml) was introduced into the anolyte compartment of a plate and frame type electrolytic cell. The anode was a platinum-clad niobium expanded mesh having both surfaces coated with 63.5 micrometer of platinum (total surface area of ca. 240 $cm^2$). The anolyte compartment was separated from the catholyte compartment by a commercial perfluorinated ion exchange membrane (Nafion®390). The anolyte compartment was maintained at a temperature of 50°C while a constant current of 14 amps was passed for 22 minutes resulting in an orange colored anolyte solution having a Ce(IV) concentration of 0.53 molar and a current efficiency of 88%. the catholyte compartment contained a stainless steel cathode and caused a clean proton reduction to hydrogen gas.

The cerium salts were at concentrations where no precipitation was observed.

Example A-1A

260 parts of methanesulfonic acid and 81 parts of cerous carbonate (obtained as the pentahydrate) were mixed according to Example A-1 above to form a 0.88M cerous methanesulfonate aqueous solution having 4M of free methanesulfonic acid. Three portions of 400 milliliters of the solution were separately introduced into an electrolytic cell as described in Example I except that the anode was a reticulated titanium metal 0.25 in. thick by 50 $cm^2$ in area (Ti-Retec® anode of Eltec Corp). The anolyte compartment was maintained at 60$^c$ for each run. The first portion was oxidized at a current of 300 mA/$cm^2$ for 40 minutes to yield 0.87 molar $Ce^{+4}$ with a current efficiency of 94%. The second portion was run at 400 mA/$cm^2$ for 30 minutes to yield 0.86M of $Ce^{+4}$ at 92 percent current efficiency. The third portion was run at 500 mA/$cm^2$ to yield 0.83M of $Ce^{+4}$ with a current efficiency of 89 percent.

Example A-1B

113.7 parts of methanesulfonic acid, 61.6 parts of cerous carbonate (obtained as the pentahydrate) and enough water to make the final volume 160 ml were mixed according to Example A-1 above to form a 1.4M cerous methanesulfonate aqueous solution having 3.2M of free methanesulfonic acid. 160 milliliters of this solution was introduced into an electrolytic cell as described in Example I, except that the anode was a flat sheet (50 $cm^2$) of platinum-clad niobium (63.5 micrometer thickness of platinum). The anolyte compartment was maintained at a temperature of 40—47°C while a constant current of 5 amps was passed for 40 min, resulting in a cerium(IV) concentration 0.75M with a current efficiency of 96%.

Example A-2

200 parts by volume of the resultant solution obtained from the electrolytic cell of Example A-1 above (0.53M Ce(IV)/5.5M $CH_3SO_3H$) was transferred to a reaction vessel and heated to 80°C under a nitrogen atmosphere. While vigorously stirring, 1.73 parts of toluene were added to the solution and stirring was continued for about 10 minutes. The resultant colorless mixture was cooled and then extracted with 3 portions of about 106 parts methylene chloride. The extracted solutions were combined and quantitatively analyzed by gas chromatography. The analysis showed a 98.5 percent conversion of toluene and 92 percent selectivity to benzaldehyde.

Examples A-3 to 19

The procedure of Example A-2 was repeated except that the organic compound and conditions were changed as defined in Table I below. The Table I also lists the percent conversion of starting compound, major product(s) recovered, and percentage selectivity.

Table I

| Example | Organic Substrate | Conc. $CH_3SO_3H$ | Temp (C) | Reaction Time (min) | Conversion (%) | Major Product(s) | Selectivity Based on Substrate % |
|---|---|---|---|---|---|---|---|
| A-3 | p-xylene | 5.5M | 80 | 12 | 98 | p-tolualdehyde | 80 |
| 4 | m-xylene | 5.5M | 60 | 10 | 91 | m-tolualdehyde | 77 |
| 5 | o-xylene | 5.5M | 60 | 40 | 98 | o-tolualdehyde | 81 |
| 6 | p-chlorotoluene | 5.5M | 80 | 15 | 99 | p-chlorobenzaldehyde | 87 |
| 7 | m-chlorotoluene | 8.5M | 80 | 9 | 82 | m-chlorobenzaldehyde | 24 |
| 8 | o-chlorotoluene | 8.5M | 80 | 9 | 92 | o-chlorobenzaldehyde | 73 |
| 9 | o-tolyl methanesulfonate | 8.5M | 80 | 10 | 98 | o-salicyl methanesulfonate | 70 |
| 10 | ethylbenzene | 5.5M | 80 | 13 | 92 | acetophenone | 48 |
| 11 | p-t-butyltoluene(a) | 5.5M | 80 | 20 | 31 | p-t-butylbenzaldehyde | 88 |
| 12 | p-ethyltoluene(a) | 3.7M | 60 | 9 | 32 | p-methylacetophenone,<br>p-ethylbenzaldehyde | 76<br>10 |
| 13 | p-isopropyltoluene(a) | 3.7M | 40 | 20 | 20 | p-methylacetophenone,<br>p-isopropylbenzaldehyde | 54<br>20 |
| 14 | styrene | 5.0M | 40 | 8 | 89 | benzaldehyde | 98 |
| 15 | 1,2,3,5-tetramethylbenzene(a) | 5.0M | 5 | 35 | 27 | 2,4,6-trimethylbenzaldehyde | 69 |
| 16 | 1,2,3,5-tetrahydronaphthalene(a) | 5.0M | 5 | 315 | 19 | 1-tetralone | 69 |
| 17 | naphthalene(b) | 2.5M | 60 | 35 | 92 | 1,4-naphthoquinone | 97 |
| 18 | 2-methylnaphthalene(b) | 2.5M | 60 | 60 | 90 | 2-methyl-1,4-naphthoquinone<br>6-methyl-1,4-naphthoquinone | 50<br>15 |
| 19 | 1-nitronaphthalene(b) | 3.7M | 60 | 90 | 75 | 5-nitro-1,4-naphthoquinone | 82 |

(a) A three-fold excess relative to Ce(IV).

(b) Substrate diluted 1:20 with 1,2 dichloroethane before oxidation.

Example A-20

23.7 parts of methanesulfonic acid was slowly added to a stirred suspension of 17.3 parts of cerous carbonate (added in the form of the pentahydrate) in 43 parts water. 76 parts methanol was added to the resulting clear solution follows by 18 parts of methansulfonic acid. The resulting 150 parts by vol. of a 2:1 methanol:water solution contained 0.5M Ce(III) methanesulfonate and 1.5 M of free methanesulfonic acid.

The solution was oxidized in a plate and frame cell as described in Example A-1 above. The anolyte temperature was maintained at 1°C while a constant current of 11 amps was passed to yield a brown solution containing 0.31 M of Ce(IV) methanesulfonate after passage of 1 F/mole charge. The system thus had a current efficiency of 62 percent.

1.5 parts by volume of p-methylanisole was added to 100 parts by volume of the solution obtained from the electrolytic cell. The solution was maintained at 5°C under nitrogen for 40 minutes with agitation. The product was extracted with methylene chloride and quantitatively determined to have 98% conversion of the p-methylanisole and selectivity of p-anisaldehyde of 72%.

Example A-21

To 75 parts by volume of the solution obtained from the electrolytic cell of Example A-1 was added 1.2 parts by volume of anthracene in 50 parts by volume of toluene at 25°C under a nitrogen atmosphere. The mixture was stirred vigorously for 40 minutes. The resulting colorless mixture was extracted with methylene chloride and quantitatively analyzed by gas chromatography. The conversion of anthracene was determined to be 98% and selectivity to anthraquinone was 95%.

Example A-22

The procedure of Example A-2 was repeated except that a large excess of 27 parts of toluene was used and the reaction time was 4 min. The analysis showed a yield of 1.4 parts benzaldehyde and 0.95 parts benzyl alcohol corresponding to 57% and 19% selectivity based on the cerium (IV).

Example A-23

An in-cell oxidation of p-t-butyltoluene was done by mixing 20 mol of p-t-butyltoluene with 200 ml of a solution of 4.6 M methanesulfonic acid and 0.8M cerium(III) methanesulfonate. The mixture was used as the anolyte of an electrochemical cell which consisted of a platinum anode (flat sheet plus 3 platinum-on niobium screens), an ion-exchange membrane (Nafion®) and a steel cathode. The anolyte was heated to 60°C and a current of 110 mA/sq cm (based on membrane area) applied to the cell until 0.15 Faradays of charged were passed. Analysis of the anolyte revealed that p-t-butylbenzaldehyde had been produced with a current efficiency of 68%, and a selectivity based on p-t-butyltoluene of 83%.

Example B-1

100 parts of trifluoromethanesulfonic was mixed with 23 parts cerous carbonate (obtained as the pentahydrate) in a small quantity of water. The resultant clear solution was diluted up with addition of water to provide a 0.55 M cerous trifluoromethanesulfonate in 2.8M trifluoromethanesulfonic acid aqueous solution. 150 parts of the solution was charged to the anodic compartment of a plate and frame type electrolytic cell. The anode was a platinum-clad niobium expanded mesh having both surfaces coated with 63.5 micrometer of platinum (total surface area of ca 240 $cm^2$). The anolyte compartment was separated from the catholyte compartment by a commercial perfluorinated ion exchange membrane (Nafion® 390). The anolyte compartment was maintained at a temperature of 50° while a constant current of 11 amps was maintained to yield 0.38M Ce(IV) trifluoromethanesulfonate after passage of 1F/mole of charge. The current efficiency was 76%.

The cerium salts were at concentrations where no precipitation was observed. 1.25 parts by volume of toluene was added to 120 parts by volume of the above solution derived from the electrolytic cell. The solution was stirred for 40 minutes at 25°C under a nitrogen atmosphere. The resultant colorless solution was cooled to 0—10°C and extracted with methylene chloride and analyzed by gas chromatography. The toluene conversion was 94% and the selectivity to benzaldehyde was 80%.

Example C-1

94 parts of methanesulfonic acid was added slowly to a stirred suspension of 61 parts of cerium(III) carbonate (obtained as pentahydrate) in 60 parts water. Upon completion of carbon dioxide evolution, additional water was added to make the total volume 150 ml. 20 parts by volume of acetonitrile as cosolvent was added and the resultant system was introduced into the anolyte compartment of a plate and frame type electrolytic cell.

The anode was a flat sheet (ca 50 $cm^2$) ruthenium oxide-titanium oxide composite on titanium. The anolyte compartment was separated from the catholyte compartment by a commercial perfluorinated ion exchange membrane (Nafion® 390). The anolyte temperature was ca 40°C and a constant current of 5 amps was passed for 30 minutes resulting in a Ce(IV) concentration of 0.37M and a current efficiency of 67%. The catholyte compartment contained a stainless steel cathode and caused a clean proton reduction to hydrogen gas.

Example C-2

100 parts by volume of 0.6M Ce(IV) methanesulfonate (a slurry) and 0.8M $CH_3SO_3H$ in water was purged with nitrogen, and then 1.28 parts of naphthalene in 30 parts by volume of 1,2-dichloroethane was added. The mixture was stirred vigorously in a 60° oil bath, and 30 parts by volume of acetonitrile was added. The reaction was complete in about 10 min. The resulting mixture was cooled and extracted with 300 parts methylene chloride. Quantitative analysis by gas chromatography showed 0.08 parts naphthalene and 1.44 parts 1,4-naphthoquinone (91.4% yield, 97.2% selectivity).

Example C-3

This example is given for comparative purposes only. The procedure of Example C-2 was repeated except that acetonitrile was not added. The solid Ce(IV) material was not consumed until 60 minutes, at which time the mixture was analyzed as in Example C-2 and found to contain 0.25 parts naphthalene and 1.07 parts naphthoquinone (67.6% yield, 83.9% selectivity) and 0.05 parts phthalic acid (2.8% yield, 2.9% selectivity). This example shows that the reaction time was about six times as long and formed the desired quinone product in a yield which was substantially lower than achieved in Example C-2.

Example C-4

100 parts by volume of 0.4M Ce(IV) methanesulfonate (a slurry) and 1.0M $CH_3SO_3H$ in water was purged with nitrogen and then 1.45 parts of p-methylanisole in 30 parts by volume of 1,2-dichloroethane was added. The mixture was stirred vigorously at 25°C, and 80 parts by volume of acetonitrile was added. After 60 minutes, the mixture was extracted with 300 parts of methylene chloride. Quantitative analysis by gas chromatography showed 0.23 part p-methylanisole and 0.96 part p-anisaldehyde (59% yield, 70% selectivity).

Example D-1

276 parts of methanesulfonic acid were added slowly to a stirred suspension of 219 parts of cerium carbonate (obtained as the pentahydrate) in 150 parts water. Upon completion of the evolution of carbon dioxide, water was added to make the volume 450 milliliters, containing 1.8M cerous methanesulfonate [$Ce(CH_3SO)_3$] and 1M of free methane sulfonic acid.

The above solution was introduced into the anolyte compartment of a plate-and-frame type electrolytic cell. The anode was a platinum-clad niobium sheet having the surface coated with 63.5 micrometers of platinum (total surface area ca 50 $cm^2$). The anolyte was separated from the catholyte compartment by a commercial perfluorinated *polyolefin* ion exchange membrane (Nafion® 390). The anolyte temperature was ca 40°C, and a constant current of 5 amps was passed for 54 min, followed by 3 amps for 211 min. A slurry resulted in the anolyte compartment. The slurry had a total cerium(IV) content of 0.53 mole (theoretical yield is 0.56; 94.6% current efficiency). The catholyte compartment contained a stainless steel cathode and caused a clean proton reduction to hydrogen gas.

The anolyte slurry was stirred overnight at ambient temperature. The solid product was separated by filtration and dried for about 15 hours at 60°C under vacuum. The recovered product was washed with 150 ml of acetonitrile and then again dried under vacuum at 60°C for about 15 hours yielding 97.3 parts of yellow solid. This yellow solid had the following elemental analysis, in agreement with $Ce(CH_2SO_3)_2(OH)_2H_2O$. Calculated: Ce, 36.65%; C, 6.28%; S, 16.75%; H, 2.62%; Found: Ce, 36.2%; C, 6.59%; S, 16.97%, H, 2.57%. Differential scanning calorimetry showed the onset of the melting slope at 195.6°C with a peak maximum of 215.5°C. The infrared spectrum (KBr pellet) is shown in Figure 1.

Example D-2

23 parts of $Ce(CH_3SO_3)_2(OH)_2H_2O$ were introduced into 120 parts of water, and stirred to dissolve the solid. The solution was then heated to 60°C. 30 parts by volume of methanesulfonic acid were added over approximately 15 seconds. The solution was purged with nitrogen and a solution of 1.28 parts of naphthalene in 30 parts by volume of 1,2-dichloroethane was added. After stirring vigorously for 45 minutes the resulting mixture was cooled and extracted with 300 parts methylene chloride.

Quantitative analysis by gas chromatography showed a 90 percent conversion of naphthalene and 90% selectivity to 1,4-naphthoquinone.

Example D-3

23 parts of $Ce(CH_2SO_3)_2O\ 2H_2O$ were introduced into 140 parts of water, and the mixture was stirred for five minutes to dissolve the solid. The solution was then heated to 60°. 65 parts by volume of methansulfonic acid were added dropwise over approximately 45 seconds. The resulting solution was purged with nitrogen, and then 2.0 parts of p-xylene was added. After stirring vigorously for 30 minutes, the resulting mixture was extracted with 300 parts methylene chloride. Quantitative analysis by gas chromatography showed a 90% conversion of p-xylene and 86% selectivity to p-tolualdehyde.

Example E-1

35 parts of an aqueous solution containing 0.5 molar concentration of thallous methanesulfonate and

63.5 Normal concentration of methanesulfonic acid was electrolyzed in a divided cell using platinum electrodes. After passage of 0.015 Faradays the solution remained clear and a corresponding amount of thallium was oxidized to Tl(III).

Example E-2

35 parts of an aqueous solution containing 0.5 molar concentration of thallous sulfate and 3.5 Normal concentration of sulfuric acid was electrolyzed in a divided cell using platinum electrodes. After passage of 0.0041 Faradays the solution became cloudy and a precipitate formed.

Example E-3

25 parts by volume of an aqueous solution having 0.24 molar thallic methanesulfonate and 2 molar free methanesulfonic acid was stirred while adding 0.416 parts of chalcone (benzylidene acetophenone) in 15 parts of 1,3-dimethoxyethane. The solution was heated to 85°C and maintained at the temperature with stirring for 18 hours. The resulting solution was extracted with 1,2-dichloroethane and then analyzed by gas chromatography. The results showed an 84% conversion of the chalcone and about 20 percent selectivity to benzil (diphenylglyoxal).

Example F-1

To 83 parts by volume of 0.73 cerium(IV) methanesulfonate and 2.8M $CH_3SO_3H$ in water was added 0.1 part of $CrO_3$ in 60 parts by volume of water. This solution was heated to 75°C and purged with nitrogen, and then 1.42 parts of 2-methylnaphthalene in 30 parts by volume of 1,2-dichloroethane were added. The mixture was stirred vigorously at 75°C and 30 parts by volume of acetonitrile were added. After 30 minutes, the mixture was extracted with 300 parts by volume of methylene chloride. Quantitative analysis by gas chromatography showed 0.11 part 2-methylnaphthalene and 0.98 part 2-methyl-1,4-naphthoquinone (65% yield based on the substrate; 54% yield based on the oxidant) and 0.26 part 6-methyl 1,4-naphthoquinone (17% yield based on the substrate; 14% yield based on oxidant). Note that the desired product was 2-methyl-1,4-naphthoquinone (menadione).

Example F-2

This example is given for comparative purposes only. The procedure of Example F-1 was repeated except that the $CrO_3$ was omitted, yielding at the end of 30 minutes, 0.09 part 2-methylnaphthalene, 0.85 part 2-methyl-1,4-naphthoquinone (55% yield based on substrate; 48% yield based on oxidant and 0.2727 part 6-methyl-1,4-naphthoquinone (18% yield based on substrate; 16% yield based on oxidant).

**Claims for the Contracting States: BE CH DE FR GB GR IT LI LU NL SE**

1. A process for forming carbonyl group containing compounds from their respective organic substrate selected from aromatic and alkylaromatic compounds comprising contacting the organic substrate with an aqueous oxidant solution, said solution containing ceric methanesulfonate in the presence of at least 1.5 molar (preferably 2 to 9 molar) concentration of free methanesulfonic acid or ceric trifluoromethanesulfonate in the presence of free trifluoromethanesulfonic acid (preferably at a concentration of at least 0.75 molarity), said solution being substantially free of extraneous anions of other acids and having at least 0.2 molar cerium concentration.

2. The process of Claim 1 wherein the oxidant solution further contains a catalytic amount of chromium.

3. An indirect electrochemical oxidation process to oxidize aromatic and alkyl aromatic compounds comprising

(a) contacting an aromatic or alkyl aromatic compound with an aqueous oxidant solution, said solution containing ceric methanesulfonate and having at least 1.5 (preferably 2 to 9) molar concentration of free methanesulfonic acid therein or ceric trifluoromethanesulfonate in the presence of free trifluoromethanesulfonic acid (preferably at a concentration of at least 0.75 molarity), said solution having all ceric and cerous ions dissolved in said solution and at a concentration of at least 0.2 molar;

(b) separating and recovering the carbonyl containing product from the solution to yield a spent solution rich in cerous salts;

(c) transferring the spent solution to an electrochemical cell to cause regeneration of a solution rich in the ceric salt; and

(d) repeating steps (a), (b) and (c).

4. A process for forming carbonyl group containing compounds from their respective organic substrates selected from aromatic and alkyl substituted aromatic compounds comprising contacting the organic substrate with ceric methanesulfonate in an aqueous system composed of water and organic cosolvents containing from 0 to about 1.3 molar concentration of free methanesulfonic acid and having at least 0.2 molar cerium concentration; said organic cosolvent being substantially inert with respect to the ceric methanesulfonate and free methanesulfonic acid and soluble in water in at least 2 weight percent.

5. The process of Claim 4 wherein the organic cosolvent is selected from a $C_1$—$C_3$ alkyl nitrile and mixtures thereof (preferably acetonitrile) or a $C_1$—$C_2$ nitroalkane and mixtures thereof.

6. An indirect electrochemical oxidation process to oxidize aromatic and alkyl substituted aromatic compounds comprising

(a) contacting the organic substrate with ceric methanesulfonate in an aqueous system composed of water and organic cosolvents containing from 0 to about 1.3 molar concentration of free methanesulfonic acid and having at least 0.2 molar cerium concentration; said organic cosolvent being substantially inert with respect to the ceric methanesulfonate and free methanesulfonic acid and soluble in water in at least 2 weight percent.

(b) separating and recovering the carbonyl containing product from the system to yield a spent system rich in cerous salts;

(c) transferring the spent system to an electrochemical cell to cause regeneration of a system rich in the ceric salt; and

(d) repeating steps (a), (b) and (c).

7. A solid product having the atomic formula $CeC_2H_{10}S_2O_9$ and the structural formula selected from $Ce(CH_3SO_3)_2O\ 2H_2O$ or $Ce(CH_3SO_3)_2(OH)_2H_2O$.

8. The product of Claim 7 wherein the product has an infrared spectrum substantially as shown in Figure 1.

9. A process for oxidizing olefins comprising contacting an olefin compound with a solution (preferably aqueous) containing thallic organosulfonate salt in the presence of an excess of the corresponding free organosulfonic acid, said organosulfonic acid having the general formula $RSO_3H$ wherein R represents methyl, trifluoromethyl, benzyl, tolyl and mixtures thereof (preferably R is methyl) and said solution being substantially free of extraneous anions of inorganic acids and having from 0.1 to 9 molar thallium concentration.

10. The process of Claim 9 wherein the aqueous solution further contains an organic solvent (preferably an organic polar solvent) for the organic substrate and said solution has a concentration of extraneous anions of from 0 to about 0.5 mole per mole of thallium ions present.

11. An indirect electrochemical oxidation process to oxidize olefins comprising

(a) contacting an olefin compound with a solution (preferably aqueous) containing thallic organosulfonate and having an excess of the corresponding free organosulfonic acid therein, said organosulfonic acid having the general formula $RSO_3H$ wherein R represents methyl, trifluoromethyl, benzyl, tolyl and mixtures thereof (preferably methyl) and said solution having substantially all thallic and thallous ions dissolved in said solution and at a concentration of from 0.1 to 9 molar;

(b) separating and recovering the oxidized product from the solution to yield a spent solution rich in thallous salts;

(c) transferring the spent solution to an electrochemical cell to cause regeneration of a solution rich in the thallic salt; and

(d) repeating steps (a), (b) and (c).

12. The process of Claim 3, 6 or 11 wherein step (a) is conducted at a temperature of from about 0°C to 100°C and the electrolysis of step (c) is conducted at a cell voltage ranging from about 2 to 20 volts with a current density of from 10 to 400 $mA/cm^2$.

**Claims for the Contracting States: AT ES**

1. A process for forming carbonyl group containing compounds from their respective organic substrate selected from aromatic and alkylaromatic compounds comprising contacting the organic substrate with an aqueous oxidant solution, said solution containing ceric methanesulfonate in the presence of at least 1.5 molar (preferably 2 to 9 molar) concentration of free methanesulfonic acid or ceric trifluoromethanesulfonate in the presence of free trifluoromethanesulfonic acid (preferably at a concentration of at least 0.75 molarity), said solution being substantially free of extraneous anions of other acids and having at least 0.2 molar cerium concentration.

2. The process of Claim 1 wherein the oxidant solution further contains a catalytic amount of chromium.

3. An indirect electrochemical oxidation process to oxidize aromatic and alkyl aromatic compounds comprising

(a) contacting an aromatic or alkyl aromatic compound with an aqueous oxidant solution, said solution containing ceric methanesulfonate and having at least 1.5 (preferably 2 to 9) molar concentration of free methanesulfonic acid therein or ceric trifluoromethanesulfonate in the presence of free trifluoromethanesulfonic acid (preferably at a concentration of at least 0.75 molarity), said solution having all ceric and cerous ions dissolved in said solution and at a concentration of at least 0.2 molar;

(b) separating and recovering the carbonyl containing product from the solution to yield a spent solution rich in cerous salts;

(c) transferring the spent solution to an electrochemical cell to cause regeneration of a solution rich in the ceric salt; and

(d) repeating steps (a), (b) and (c).

4. A process for forming carbonyl group containing compounds from their respective organic substrates selected from aromatic and alkyl substituted aromatic compounds comprising contacting the

organic substrate with ceric methanesulfonate in an aqueous system composed of water and organic cosolvents containing from 0 to about 1.3 molar concentration of free methanesulfonic acid and having at least 0.2 molar cerium concentration; said organic cosolvent being substantially inert with respect to the ceric methanesulfonate and free methanesulfonic acid and soluble in water in at least 2 weight percent.

5. The process of Claim 4 wherein the organic cosolvent is selected from a $C_1$—$C_3$ alkyl nitrile and mixtures thereof (preferably acetonitrile) or a $C_1$—$C_2$ nitroalkane and mixtures thereof.

6. An indirect electrochemical oxidation process to oxidize aromatic and alkyl substituted aromatic compounds comprising

(a) contacting the organic substrate with ceric methanesulfonate in an aqueous system composed of water and organic cosolvents containing from 0 to about 1.3 molar concentration of free methanesulfonic acid and having at least 0.2 molar cerium concentration; said organic cosolvent being substantially inert with respect to the ceric methanesulfonate and free methanesulfonic acid and soluble in water in at least 2 weight percent.

(b) separating and recovering the carbonyl containing product from the system to yield a spent system rich in cerous salts;

(c) transferring the spent system to an electrochemical cell to cause regeneration of a system rich in the ceric salt; and

(d) repeating steps (a), (b) and (c).

7. A process for preparing a solid product having the atomic formula $CeC_2H_{10}S_2O_9$ and the structural formula selected from $Ce(CH_3SO_3)_2O\ 2H_2O$ or $Ce(CH_3SO_3)_2(OH)_2H_2O$ comprising contacting, in an aqueous solution, cerous oxide, carbonate, hydroxide or mixtures thereof with a small excess of free methanesulfonic acid over that required to neutralize the initial cerous salt, oxidizing said solution by electrolysis and recovering the precipitated solid product.

8. The process of claim 7, wherein the product has an infrared spectrum substantially as shown in Figure 1.

9. A process for oxidizing olefins comprising contacting an olefin compound with a solution (preferably aqueous) containing thallic organosulfonate salt in the presence of an excess of the corresponding free organosulfonic acid, said organosulfonic acid having the general formula $RSO_3H$ wherein R represents methyl, trifluoromethyl, benzyl, tolyl and mixtures thereof (preferably R is methyl) and said solution being substantially free of extraneous anions of inorganic acids and having from 0.1 to 9 molar thallium concentration.

10. The process of Claim 9 wherein the aqueous solution further contains an organic solvent (preferably an organic polar solvent) for the organic substrate and said solution has a concentration of extraneous anions of from 0 to about 0.5 mole per mole of thallium ions present.

11. An indirect electrochemical oxidation process to oxidize olefins comprising

(a) contacting an olefin compound with a solution (preferably aqueous) containing thallic organosulfonate and having an excess of the corresponding free organosulfonic acid therein, said organosulfonic acid having the general formula $RSO_3H$ wherein R represents methyl, trifluoromethyl, benzyl, tolyl and mixtures thereof (preferably methyl) and said solution having substantially all thallic and thallous ions dissolved in said solution and at a concentration of from 0.1 to 9 molar;

(b) separating and recovering the oxidized product from the solution to yield a spent solution rich in thallous salts;

(c) transferring the spent solution to an electrochemical cell to cause regeneration of a solution rich in the thallic salt; and

(d) repeating steps (a), (b) and (c).

12. The process of Claim 3, 6 or 11 wherein step (a) is conducted at a temperature of from about 0°C to 100°C and the electrolysis of step (c) is conducted at a cell voltage ranging from about 2 to 20 volts with a current density of from 10 to 400 $mA/cm^2$.

**Patentansprüche für die Vertragsstaaten: BE CH DE FR GB GR IT LI LU NL SE**

1. Verfahren zur Bildung carbonylgruppenhaltiger Verbindungen aus ihrem jeweiligen organischen Substrat, ausgewählt aus aromatischen und alkylaromatischen Verbindungen, durch Inberührungbringen des organischen Substrats mit einer wässrigen oxidierenden Lösung, wobei die Lösung Cer-IV-methansulfonat in Gegenwart einer wenigstens 1,5 molaren (vorzugsweise 2- bis 9-molaren) Konzentration an freier Methansulfonsäure oder Cer-IV-trifluormethansulfonat in Gegenwart von freier Trifluormethansulfonsäure (vorzugsweise in einer Konzentration mit einer Molarität von wenigstens 0,75) enthält, die Lösung im wesentlichen frei von fremden Anionen anderer Säuren ist und wenigstens 0,2 molar in der Cer-IV-konzentration ist.

2. Verfahren nach Anspruch 1, worin die oxidierende Lösung weiterhin eine katalytische Menge Chrom enthält.

3. Indirektes elektrochemisches Oxidationsverfahren zur Oxidation von aromatischen und alkylaromatischen Verbindungen durch

(a) Inberührungbringen einer aromatischen oder alkylaromatischen Verbindung mit einer wässrigen

oxidierenden Lösung, wobei die Lösung Cer-IV-methansulfonat enthält und wenigstens eine 1,5- (vorzugsweise 2- bis 9-) molare Konzentration an freier Methansulfonsäure darin oder Cer-IV-trifluormethansulfonat in Gegenwart von freier Trifluormethansulfonsäure (vorzugsweise in einer Konzentration mit einer Molarität von wenigstens 0,75) enthält, und die Lösung alle Cer-IV- und Cer-IIIionen in sich gelöst aufweist bei einer Konzentration mit einer Molarität von wenigstens 0,2;

(b) Abtrennen und Gewinnen des carbonylhaltigen Produkts aus der Lösung unter Erhalt einer an Cer-III-salzen reichen verbrauchten Lösung;

(c) Transferieren der verbrauchten Lösung zu einer elektrochemischen Zelle, um die Regenerierung einer an Cer-IV-salz reichen Lösung zu bewirken; und

(d) Wiederholen der Schritte (a), (b) und (c).

4. Verfahren zur Bildung carbonylgruppenhaltiger Verbindungen aus ihren entsprechenden organischen Substraten, ausgewählt aus aromatischen und alkylsubstituierten aromatischen Verbindungen, durch Inberührungbringen des organischen Substrats mit Cer-IV-methansulfonat in einem wässrigen System, das aus Wasser und organischen Kolösungsmitteln mit einer O-bis etwa 1,3-molaren Konzentration an freier Methansulfonsäure zusammengesetzt ist und eine wenigstens 0,2-molare Cerkonzentration aufweist, wobei das organische Kolösungsmittel im wesentlichen inert hinsichtlich des Cer-IVmethansulfonats und der freien Methansulfonsäure ist und in Wasser zu wenigstens 2 Gew.-% löslich ist.

5. Verfahren nach Anspruch 4, worin das organische Kolösungsmittel aus einem $C_1$—$C_3$-Alkylnitril und Mischungen davon (vorzugsweise Acetonitril) oder einem $C_1$—$C_2$-Nitroalkan und Mischungen davon ausgewählt ist.

6. Indirektes electrochemisches Oxidationsverfahren zur Oxidation von aromatischen und alkylsubstituierten aromatischen Verbindungen durch

.(a) Inberührungbringen des organischen Substrats mit Cer-IV-methansulfonat in einem wässrigen System, das aus Wasser und organischen Kolösungsmitteln mit einer O-bis etwa 1,3-molaren Konzentration an freier Methansulfonsäure zusammengesetzt ist und eine wenigstens 0,2-molare Cerkonzentration aufweist, wobei das organische Kolösungsmittel im wesentlichen inert hinsichtlich des Cer-IVmethansulfonats und der freien Methansulfonsäure ist und in Wasser zu wenigstens 2 Gew.-% löslich ist;

(b) Abtrennen und Gewinnen des carbonylhaltigen Produkts aus dem System unter Erhalt eines an Cer-III-salzen reichen verbrauchten Systems;

(c) Transferieren des verbrauchten Systems zu einer elektrochemischen Zelle, um die Regenerierung eines an Cer-IV-salzen reichen Systems zu bewirken; und

(d) Wiederholen der Schritte (a), (b) und (c).

7. Festes Produkt mit der Atomzusammensetzung $CeC_2H_{10}S_2O_9$ und der Strukturformel, ausgewählt aus $Ce(CH_3SO_3)_2O\ 2H_2O$ oder $Ce(CH_3SO_3)_2(OH)_2H_2O$.

8. Produkt nach Anspruch 7, worin das Produkt im wesentlichen das in Figur 1 gezeigte Infrarotspektrum hat.

9. Verfahren zur Oxidation von Olefinen durch Inberührungbringen einer Olefinverbindung mit einer Lösung, (vorzugsweise einer wässrigen), die Thallium-III-organosulfonatsalz in Gegenwart einer Überschusses der entsprechenden freien Organosulfonsäure enthält, wobei die Organosulfonsäure die allgemeine Formel $RSO_3H$ aufweist, worin R Methyl-, Tri-fluormethyl-, Benzyl-, Tolyl und Mischungen davon darstellt (vorzugsweise ist R Methyl), und die Lösung im wesentlichen frei von fremden Anionen anorganischer Säuren ist und eine 0,1- bis 9-molare Thalliumkonzentration aufweist.

10. Verfahren nach Anspruch 9, worin die wässrige Lösung weiterhin ein organisches Lösungsmittel (vorzugsweise ein polares organisches Lösungsmittel) für das organische Substrat enthält und die Lösung eine Konzentration an fremden Anionen von 0 bis etwa 0,5 Mol pro Mol an vorhandenen Thalliumionen aufweist.

11. Indirektes elektrochemisches Oxidationsverfahren zur Oxidation von Olefinen durch

(a) Inberührungbringen einer Olefinverbindung mit einer Lösung (vorzugsweise einer wässrigen), die Thallium-III-organosulfonat enthält und einen Überschuß der entsprechenden freien Organosulfonsäure darin aufweist, wobei die Organosulfonsäure die allgemeine Formel $RSO_3H$ aufweist, worin R Methyl, Trifluormethyl, Benzyl, Tolyl und Mischungen davon (vorzugsweise Methyl) darstellt, und die Lösung im wesentlichen alle Thallium-III- und Thallium-I-ionen in sich gelöst enthält bei einer Konzentration mit einer Molarität von 0,1 bis 9;

(b) Abtrennen und Gewinnen des oxidierten Produkts aus der Lösung unter Erhalt einer an Thallium-I-salzen reichen verbrauchten Lösung;

(c) Transferieren der verbrauchten Lösung zu einer elektrochemischen Zelle, um die Regenerierung einer an Thallium-III-salzen reichen Lösung zu bewirken; und

(d) Wiederholen der Schritte (a), (b) und (c).

12. Verfahren nach Anspruch 3, 6 oder 11, worin Schritt (a) bei einer Temperatur von etwa 0°C bis 100°C durchgeführt wird und die Elektrolyse in Schritt (c) bei einer Zellspannung im Bereich von etwa 2 bis 20 V mit einer Stromdichte von 10 bis 400 $mA/cm^2$ durchgeführt wird.

**Patentansprüche für die Vertragsstaaten: AT ES**

1. Verfahren zur Bildung carbonylgruppenhaltiger Verbindungen aus ihrem jeweiligen organischen Substrat, ausgewählt aus aromatischen und alkylaromatischen Verbindungen, durch Inberührungbringen des organischen Substrats mit einer wässrigen oxidierenden Lösung, wobei die Lösung Cer-IV-methansulfonat in Gegenwart einer wenigstens 1,5 molaren (vorzugsweise 2- bis 9-molaren) Konzentration an freier Methansulfonsäure oder Cer-IV-trifluormethansulfonat in Gegenwart von freier Trifluormethansulfonsäure (vorzugsweise in einer Konzentration mit einer Molarität von wenigstens 0,75) enthält, die Lösung im wesentlichen frei von fremden Anionen anderer Säuren ist und wenigstens 0,2 molar in der Cer-IV-konzentration ist.

2. Verfahren nach Anspruch 1, worin die oxidierende Lösung weiterhin eine katalytische Menge Chrom enthält.

3. Indirektes elektrochemisches Oxidationsverfahren zur Oxidation von aromatischen und alkylaromatischen Verbindungen durch

(a) Inberührungbringen einer aromatischen oder alkylaromatischen Verbindung mit einer wässrigen oxidierenden Lösung, wobei die Lösung Cer-IV-methansulfonat enthält und wenigstens eine 1,5-(vorzugsweise 2- bis 9-) molare Konzentration an freier Methansulfonsäure darin oder Cer-IV-trifluormethansulfonat in Gegenwart von freier Trifluormethansulfonsäure (vorzugsweise in einer Konzentration mit einer Molarität von wenigstens 0,75) enthält, und die Lösung alle Cer-IV- und Cer-IIIionen in sich gelöst aufweist bei einer Konzentration mit einer Molarität von wenigstens 0,2;

(b) Abtrennen und Gewinnen des carbonylhaltigen Produkts aus der Lösung unter Erhalt einer an Cer-III-salzen reichen verbrauchten Lösung;

(c) Transferieren der verbrauchten Lösung zu einer elektrochemischen Zelle, um die Regenerierung einer an Cer-IV-salz reichen Lösung zu bewirken; und

(d) Wiederholen der Schritte (a), (b) und (c).

4. Verfahren zur Bildung carbonylgruppenhaltiger Verbindungen aus ihren entsprechenden organischen Substraten, ausgewählt aus aromatischen und alkylsubstituierten aromatischen Verbindungen, durch Inberührungbringen des organischen Substrats mit Cer-IV-methansulfonat in einem wässrigen System, das aus Wasser und organischen Kolösungsmitteln mit einer O-bis etwa 1,3-molaren Konzentration an freier Methansulfonsäure zusammengesetzt ist und eine wenigstens 0,2-molar Cerkonzentration aufweist, wobei das organische Kolösungsmittel im wesentlichen inert hinsichtlich des Cer-IVmethansulfonats und der freien Methansulfonsäure ist und in Wasser zu wenigstens 2 Gew.-% löslich ist.

5. Verfahren nach Anspruch 4, worin das organische Kolösungsmittel aus einem $C_1$—$C_3$-Alkylnitril und Mischungen davon (vorzugsweise Acetonitril) oder einem $C_1$—$C_2$-Nitroalkan und Mischungen davon ausgewählt ist.

6. Indirektes electrochemisches Oxidationsverfahren zur Oxidation von aromatischen und alkylsubstituierten aromatischen Verbindungen durch

(a) Inberührunbringen des organischen Substrats mit Cer-IV-methansulfonat in einem wässrigen System, das aus Wasser und organischen Kolösungsmitteln mit einer O-bis etwa 1,3-molaren Konzentration an freier Methansulfonsäure zusammengesetzt ist und eine wenigstens 0,2-molare Cerkonzentration aufweist, wobei das organische Kolösungsmittel im wesentlichen inert hinsichtlich des Cer-IVmethansulfonats und der freien Methansulfonsäure ist und in Wasser zu wenigstens 2 Gew.-% löslich ist;

(b) Abtrennen und Gewinnen des carbonylhaltigen Produkts aus dem System unter Erhalt eines an Cer-III-salzen reichen verbrauchten Systems;

(c) Transferieren des verbrauchten Systems zu einer elektrochemischen Zelle, um die Regenerierung eines an Cer-IV-salzen reichen Systems zu bewirken; und

(d) Wiederholen der Schritte (a), (b) und (c).

7. Verfahren zur Herstellung eines festen Produkts mit der Atomzusammensetzung $CeC_2H_{10}S_2O_9$ und der Strukturformel, ausgewählt aus $Ce(CH_3SO_3)_2O\ 2H_2O$ oder $Ce(CH_3SO_3)_2(OH)_2H_2O$ durch Inberührungbringen von Cer-III-oxid, -carbonat, -hydroxid oder Mischungen davon in einer wässrigen Lösung mit einem kleinen Überschuß an freier Methansulfonsäure über die zur Neutralisation des anfänglichen Cer-III-salzes benötigte Menge, Oxidieren der Lösung durch Elektrolyse und Gewinnen des ausgefällten festen Produkts.

8. Verfahren nach Anspruch 7, worin das Produkt im wesentlichen das in Figur 1 gezeigte Infrarotspektrum hat.

9. Verfahren zur Oxidation von Olefinen durch Inberührungbringen einer Olefinverbindung mit einer Lösung, (vorzugsweise einer wässrigen), die Thallium-III-organosulfonatsalz in Gegenwart eines Überschusses der entsprechenden freien Organosulfonsäure enthält, wobei die Organosulfonsäure die allgemeine Formel $RSO_3H$ aufweist, worin R Methyl-, Tri-fluormethyl-, Benzyl-, Tolyl und Mischungen davon darstellt (vorzugsweise ist R Methyl), und die Lösung im wesentlichen frei von fremden Anionen anorganischer Säuren ist und eine 0,1- bis 9-molare Thalliumkonzentration aufweist.

10. Verfahren nach Anspruch 9, worin die wässrige Lösung weiterhin ein organisches Lösungsmittel (vorzugsweise ein polares organisches Lösungsmittel) für das organische Substrat enthält und die Lösung eine Konzentration an fremden Anionen von 0 bis etwa 0,5 Mol pro Mol an vorhandenen Thalliumionen aufweist.

11. Indirektes elektrochemisches Oxidationsverfahren zur Oxidation von Olefinen durch

(a) Inberührungbringen einer Olefinverbindung mit einer Lösung (vorzugsweise einer wässrigen), die Thallium-III-organosulfonat enthält und einen Überschuß der entsprechenden freien Organosulfonsäure darin aufweist, wobei die Organosulfonsäure die allgemeine Formel $RSO_3H$ aufweist, worin R Methyl, Trifluormethyl, Benzyl, Tolyl und Mischungen davon (vorzugsweise Methyl) darstellt, und die Lösung im wesentlichen alle Thallium-III- und Thallium-I-ionen in sich gelöst enthält bei einer Konzentration mit einer Molarität von 0,1 bis 9;

(b) Abtrennen und Gewinnen des oxidierten Produkts aus der Lösung unter Erhalt einer an Thallium-I-salzen reichen verbrauchten Lösung;

(c) Transferieren der verbrauchten Lösung zu einer elektrochemischen Zelle, um die Regenerierung einer an Thallium-III-salzen reichen Lösung zu bewirken; und

(d) Wiederholen der Schritte (a), (b) und (c).

12. Verfahren nach Anspruch 3, 6 oder 11, worin Schritt (a) bei einer Temperatur von etwa 0°C bis 100°C durchgeführt wird und die Elektrolyse in Schritt (c) bei einer Zellspannung im Bereich von etwa 2 bis 20 V mit einer Stromdichte von 10 bis 400 mA/cm$^2$ durchgeführt wird.

**Revendications pour les Etats contractants: BE CH DE FR GB GR IT LI LU NL SE**

1. Procédé pour la formation de composés contenant un groupe carbonyle à partir de leur substrat organique respectif choisi parmi des composés aromatiques et alkyles aromatiques comprenant la mise en contact du substrat organique avec une solution aqueuse d'un oxydant, ladite solution contenant du méthanesulfonate cérique, en présence d'une concentration à au moins 1,5 molaires (de préférence 2 à 9 molaires) de l'acide méthanesulfonique libre ou du trifluorométhanesulfonate cérique en présence d'acide trifluorométhanesulfonique libre (de préférence à une concentration d'une molarité d'au moins 0,75), ladite solution étant sensiblement exempte d'anions étrangers d'autres acides et ayant une concentration en cérium d'au moins 0,2 molaire.

2. Procédé de la revendication 1 où la solution de l'oxydant contient de plus une quantité catalytique de chrome.

3. Procédé d'oxydation électrochimique indirecte pour oxyder des composés aromatiques et alkyles aromatiques comprenant:

(a) la mise en contact d'un composé aromatique ou alkyle aromatique avec une solution aqueuse d'un oxyde, ladite solution contenant du méthanesulfonate cérique et ayant une concentration molaire d'au moins 1,5 (de préférence 2 à 9) de l'acide méthanesulfonique libre ou de l'acide trifluorométhanesulfonique cérique en présence d'acide trifluorométhanesulfonique libre (de préférence à une concentration d'un molarité d'au moins 0,75), ladite solution ayant tous les ions cériques et céreux dissous dans ladite solution et à une concentration d'au moins 0,2 molaire;

(b) la séparation et la récupération du produit contenant un carbonyle de la solution pour donner une solution usée riche en sels céreux;

(c) le transfert de la solution usée à une cellule électrochimique pour provoquer la régénération d'une solution riche en sel cérique; et

(d) la répétition des étapes (a), (b) et (c).

4. Procédé de formation de composés contenant un groupe carbonyle à partir de leurs substrats organiques respectifs choisis parmi des composés aromatiques et aromatiques alkyles-substitués comprenant la mise en contact du substrat organique avec du méthanesulfonate cérique dans un système aqueux composé d'eau et de cosolvants organiques contenant une concentration molaire de O à environ 1,3 de l'acide méthanesulfonique libre et ayant une concentration en cérium d'au moins 0,2 molaire; ledit cosolvant organique étant sensiblement inerte par rapport au méthanesulfonate cérique et à l'acide méthanesulfonique libre et soluble dans l'eau à au moins 2% en poids.

5. Procédé de la revendication 4 où le cosolvant organique est choisi parmi un alkyl nitrile $C_1$—$C_3$ et leurs mélanges (de préférence l'acétonitrile) ou un nicroalcane $C_1$—$C_2$ et leurs mélanges.

6. Procédé d'oxydation électrochimique indirecte pour oxyder des composés aromatiques et aromatiques alkyles-substitués comprenant

(a) la mise en contact du substrat organique avec du méthanesulfonate cérique dans un système aqueux composé d'eau et de cosolvants organiques contenant une concentration molaire de O à environ 1,3 de l'acide méthanesulfonique libre et ayant une concentration en cérium d'au moins 0,2 molaire; ledit cosolvant organique étant sensiblement inerte par rapport au méthanesulfonate cérique et exempt d'acide méthanesulfonique libre et soluble dans l'eau à au moins 2% en poids.

(b) la séparation et la récupération du produit contenant un carbonyle du système pour donner un système usé riche en sels cériques;

(c) le transfert du système uné à une cellule électrochimique pour provoquer la régénération d'un système riche en sel cérique; et

(d) la répétition des étapes (a), (b) et (c).

7. Produit solide ayant la formule atomique $CeC_2H_{10}S_2O_9$ et la formule de structure choisie parmi $Ce(CH_3SO_3)_2O\ 2H_2O$ ou $Ce(CH_3SO_3)_2(OH)_2H_2O$.

8. Produit de la revendication 7 où le produit a un spectre infrarouge sensiblement tel que montré à la figure 1.

9. Procédé pour l'oxydation d'oléfines comprenant la mise en contact d'un composé d'oléfine avec une solution (de préférence aqueuse) contenant un sel d'organosulfonate thallique en présence d'un excès de l'acide organosulfonique libre correspondant, ledit acide organosulfonique ayant la formule générale $RSO_3H$ où R représente méthyle, trifluorométhyle, benzyle, tolyle et leurs mélanges (de préférence R est méthyle) et ladite solution étant sensiblement exempte d'anions étrangers d'acides inorganiques et ayant une concentration en thallium de 0,1 à 9 molaires.

10. Procédé de la revendication 9 où la solution aqueuse contient de plus un solvant organique (de préférence un solvant polaire organique) pour le substrat organique et ladite solution a une concentration en anions étrangers de 0 à environ 0,5 mole par mole des ions présents de thallium.

11. Procédé d'oxydation électrochimique indirecte pour oxyder des oléfines comprenant

(a) la mise en contact d'une composé d'oléfine avec une solution (de préférence aqueuse) contenant un organosulfonate thallique et contenant un excès de l'acide organosulfonique libre correspondant, ledit acide organosulfonique ayant la formule générale $RSO_3H$ où R représente méthyl, trifluorométhyle, benzyle, tolyle et leurs mélanges (de préférence méthyle) et ladite solution ayant sensiblement tous les ions thalliques et thalleux dissous dans ladite solution à une concentration molaire de 0,1 à 9;

(b) la séparation et la récupération du produit oxydé de la solution pour donner une solution usée riche en sels thalleux;

(c) le transfert de la solution usée à une cellule électrochimique pour provoquer la régénération d'une solution riche en sel thallique; et

(d) la répétition des étapes (a), (b) et (c).

12. Procédé de la revendication 3, 6 ou 11 où l'étape (a) est entreprise à une température d'environ 0°C à 100°C et l'électrolyse de l'étape (c) est entreprise à une tension de la cellule qui est comprise entre environ 2 et 20 volts avec une densité de courant de 10 à 400 $mA/cm^2$.

**Revendications pour les Etats contractants: AT ES**

1. Procédé pour la formation de composés contenant un groupe carbonyle à partir de leur substrat organique respectif choisi parmi des composés aromatiques et alkyles aromatiques comprenant la mise en contact du substrat organique avec une solution aqueuse d'un oxydant, ladite solution contenant du méthanesulfonate cérique, en présence d'une concentration à au moins 1,5 molaires (de préférence 2 à 9 molaires) de l'acide méthanesulfonique libre ou du trifluorométhanesulfonate cérique en présence d'acide trifluorométhanesulfonique libre (de préférence à une concentration d'une molarité d'au moins 0,75), ladite solution étant sensiblement exempte d'anions étrangers d'autres acides et ayant une concentration en cérium d'au moins 0,2 molaire.

2. Procédé de la revendication 1 où la solution de l'oxydant contient de plus une quantité catalytique de chrome.

3. Procédé d'oxydation électrochimique indirecte pour oxyder des composés aromatiques et alkyles aromatiques comprenant:

(a) la mise en contact d'un composé aromatique ou alkyle aromatique avec une solution aqueuse d'un oxyde, ladite solution contenant du méthanesulfonate cérique et ayant une concentration molaire d'au moins 1,5 (de préférence 2 à 9) de l'acide méthanesulfonique libre ou de l'acide trifluorométhanesulfonique cérique en présence d'acide trifluorométhanesulfonique libre (de préférence à une concentration d'un molarité d'au moins 0,75), ladite solution ayant tous les ions cériques et céreux dissous dans ladite solution et à une concentration d'au moins 0,2 molaire;

(b) la séparation et la récupération du produit contenant un carbonyle de la solution pour donner une solution usée riche en sels céreux;

(c) le transfert de la solution usée à une cellule électrochimique pour provoquer la régénération d'une solution riche en sel cérique; et

(d) la répétition des étapes (a), (b) et (c).

4. Procédé de formation de composés contenant un groupe carbonyle à partir de leurs substrats organiques respectifs choisis parmi des composés aromatiques et aromatiques alkyles-substitués comprenant la mise en contact du substrat organique avec du méthanesulfonate cérique dans un système aqueux composé d'eau et de cosolvants organiques contenant une concentration molaire de O à environ 1,3 de l'acide méthanesulfonique libre et ayant une concentration en cérium d'au moins 0,2 molaire; ledit cosolvant organique étant sensiblement inerte par rapport au méthanesulfonate cérique et à l'acide méthanesulfonique libre et soluble dans l'eau à au moins 2% en poids.

5. Procédé de la revendication 4 où le cosolvant organique est choisi parmi un alkyl nitrile $C_1$—$C_3$ et leurs mélanges (de préférence l'acétonitrile) ou un nitroalcane $C_1$—$C_2$ et leurs mélanges.

6. Procédé d'oxydation électrochimique indirecte pour oxyder des composés aromatiques et aromatiques alkyles-substitués comprenant

(a) la mise en contact du substrat organique avec du méthanesulfonate cérique dans un système aqueux composé d'eau et de cosolvants organiques contenant une concentration molaire de O à environ

1,3 de l'acide méthanesulfonique libre et ayant une concentration en cérium d'au moins 0,2 molaire; ledit cosolvant organique étant sensiblement inerte par rapport au méthanesulfonate cérique et exempt d'acide méthanesulfonique libre et soluble dans l'eau à au moins 2% en poids.

(b) la séparation et la récupération du produit contenant un carbonyle du système pour donner un système usé riche en sels cériques;

(c) le transfert du système uné à une cellule électrochimique pour provoquer la régénération d'un système riche en sel cérique; et

(d) la répétition des étapes (a), (b) et (c).

7. Procédé de préparation d'un produit solide ayant la formule atomique $CeC_2H_{10}S_2O_9$ et la formule de structure choisie parmi $Ce(CH_3SO_3)_2O\ 2H_2O$ ou $Ce(CH_3SO_3)_2(OH)_2H_2O$ comprenant la mise en contact, dans une solution aqueuse, d'oxyde, carbonate, hydroxyde céreux ou leur mélange avec un petit excès d'acide méthane sulfonique, au delà de ce qui est requis pour neutraliser le sel céreux initial, l'oxydation de ladite solution par électrolyse et récupération du produit solide précipité.

8. Procédé de la revendication 7 où le produit a un spectre infrarouge sensiblement tel que représenté à la figure 1.

9. Procédé pour l'oxydation d'oléfines comprenant la mise en contact d'un composé d'oléfine avec une solution (de préférence aqueuse) contenant un sel d'organosulfonate thallique en présence d'un excès de l'acide organosulfonique libre correspondant, ledit acide organosulfonique ayant la formule générale $RSO_3H$ où R représente méthyle, trifluorométhyle, benzyle, tolyle et leurs mélanges (de préférence R est méthyle) et ladite solution étant sensiblement exempte d'anions étrangers d'acides inorganiques et ayant une concentration en thallium de 0,1 à 9 molaires.

10. Procédé de la revendication 9 où la solution aqueuse contient de plus un solvant organique (de préférence un solvant polaire organique) pour le substrat organique et ladite solution a une concentration en anions étrangers de 0 à environ 0,5 mole par mole des ions présents de thallium.

11. Procédé d'oxydation électrochimique indirecte pour oxyder des oléfines comprenant

(a) la mise en contact d'une composé d'oléfine avec une solution (de préférence aqueuse) contenant un organosulfonate thallique et contenant un excès de l'acide organosulfonique libre correspondant, ledit acide organosulfonique ayant la formule générale $RSO_3H$ où R représente méthyl, trifluorométhyle, benzyle, tolyle et leurs mélanges (de préférence méthyle) et ladite solution ayant sensiblement tous les ions thalliques et thalleux dissous dans ladite solution à une concentration molaire de 0,1 à 9;

(b) la séparation et la récupération du produit oxydé de la solution pour donner une solution usée riche en sels thalleux;

(c) le transfert de la solution usée à une cellule électrochimique pour provoquer la régénération d'une solution riche en sel thallique; et

(d) la répétition des étapes (a), (b) et (c).

12. Procédé de la revendication 3, 6 ou 11 où l'étape (a) est entreprise à une température d'environ 0°C à 100°C et l'électrolyse de l'étape (c) est entreprise à une tension de la cellule qui est comprise entre environ 2 et 20 volts avec une densité de courant de 10 à 400 $mA/cm^2$.

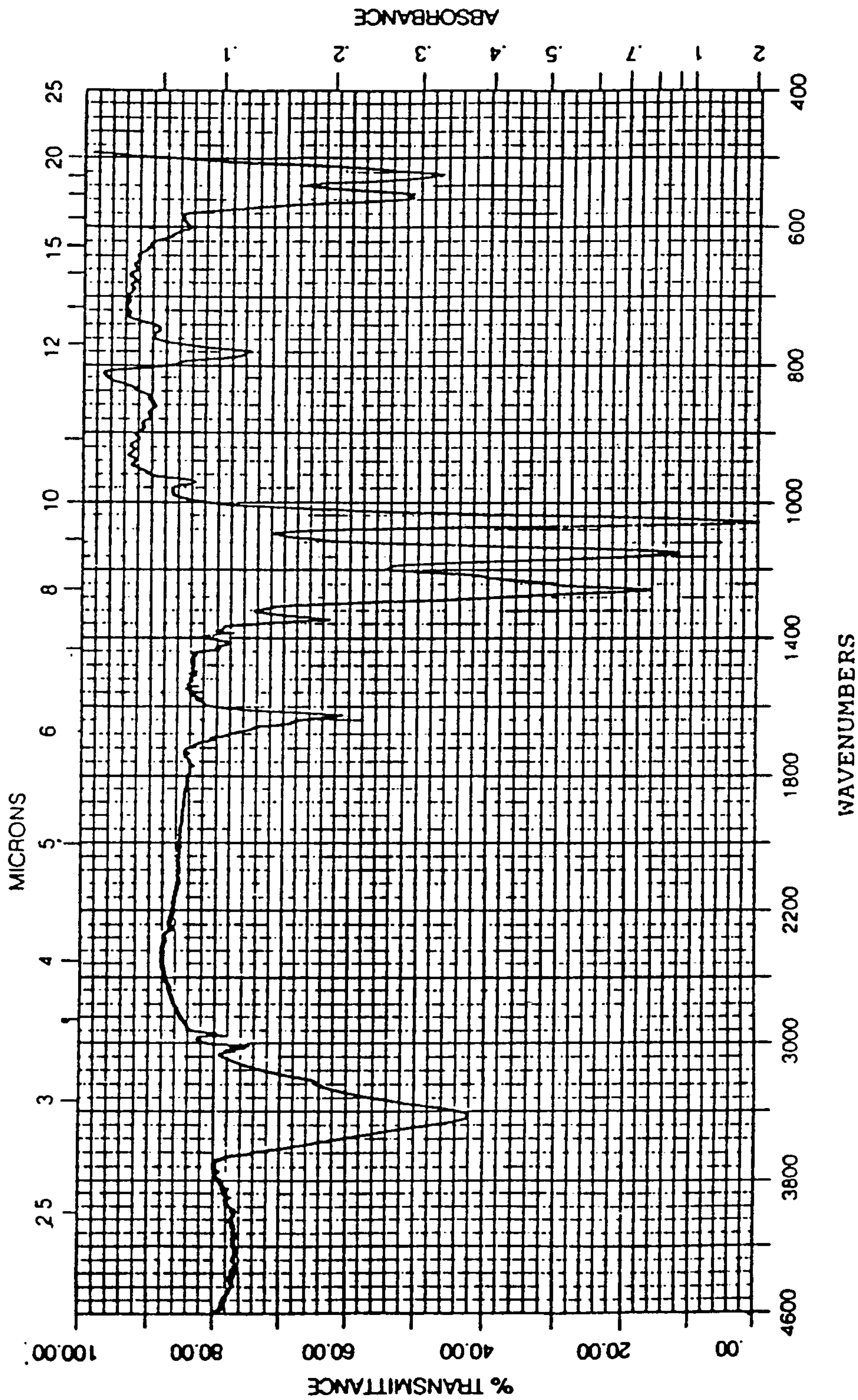


FIG.I